# EUROPEAN PATENT APPLICATION

(11) **EP 2 818 559 A1**
(43) Date of publication of application: **31.12.2014**
(21) Application number: 13751837.9
(22) Date of filing: 20.02.2013
(51) Int. Cl.: C12Q 1/34, C12Q 1/00, C12Q 1/26

(54) **ENZYME ASSAY METHOD**

(30) Priority: 20.02.2012 JP 2012034045
(71) Applicant: Japan Agency for Marine-Earth Science and Technology, Yokosuka-shi, Kanagawa 237-0061 (JP); Kyokuto Pharmaceutical Industrial Co., Ltd., Chuo-ku Tokyo 103-0024 (JP)
(72) Inventor: DEGUCHI, Shigeru, Yokosuka-shi Kanagawa 237-0061 (JP); TSUDOME, Mikiko, Yokosuka-shi Kanagawa 237-0061 (JP); NAGAKI, Kazunori, Takahagi-shi Ibaraki 318-0004 (JP); TODAKA, Nemuri, Takahagi-shi Ibaraki 318-0004 (JP); KUROSAWA, Yasunori, Takahagi-shi Ibaraki 318-0004 (JP)
(74) Representative: Michalski Hüttermann & Partner Patentanwälte mbB
(86) International application number: PCT/JP2013/054184
(87) International publication number: WO 2013/125579

(57) **Abstract**

Providing is a new enzyme assay method for enzymes having a water-insoluble or substantially water-insoluble substrate. In the method for measuring enzymatic activity, a prescribed amount of an enzyme is disposed on a part of the surface of a gel comprising dispersoids, at least some of which are the substrate of the enzyme. Recesses formed in the surface of the gel by the action of the enzyme are measured, and the enzyme activity is calculated on the basis of the measurement results and the amount of the enzyme. The measurement of the recesses formed in the surface of the gel is performed using a method for measuring the shape and the volume of the recesses, a method for measuring changes in the optical transmittance of the gel due to the formation of the recesses, or a method for measuring changes in the optical reflectance of the gel surface due to the formation of the recesses.

## Description

### [Cross-Reference to Related Application]

This application claims benefit of priority to Japanese Patent Application No. 2012-34045 filed on February 20, 2012, which is expressly incorporated herein by reference in its entirety.

### [Technical Field]

The present invention relates to a method for measuring the activity of an enzyme on a substrate that is insoluble or of low solubility in water.

### [Background Art]

The activity of an enzyme is normally measured by dissolving or dispersing a substrate in water, subjecting it to the action of the enzyme, and using some form of chromatography, spectroscopic technique, or the like to measure a compound that is produced.

However, for enzymes acting on water-insoluble substrates such as cellulose, the above technique cannot be readily applied because the substrate is insoluble in water. Since the applied reaction of the enzyme on the substrate dispersed in water takes place in a heterogeneous system, there are problems in that the reaction rate is slow, the reaction rate is greatly affected by the structural characteristics of the substrate (such as the degree of crystallization and the specific surface area), and detection sensitivity is low.

Instead of the above method, there is a method of measuring enzymatic activity by determining the amount of reduction in mass generated by the hydrolysis of a water-insoluble substrate by an enzyme (Nonpatent Reference 1).

[Patent Reference 1] WO2005/083056
[Nonpatent Reference 1] S. Ahola, X. Turon, M. Osterberg, J. Laine, O. J. Rojas, Langmuir 24, 11592-11599 (2008).
[Nonpatent Reference 2] S. Deguchi, M. Tsudome, Y. Shen, S. Konishi, K. Tsujii, S. Ito, K. Horikoshi, Soft Matter, 3 (9), 1170-1175 (2007).
[Nonpatent Reference 3] S. Hirano, R. Yamaguchi, Biopolymers 15, 1685-1691 (1976).

Patent Reference 1 and Nonpatent References 1 to 3 are expressly incorporated herein by reference in their entirety.

### [Summary of the Invention]

The method described in Nonpatent Reference 1 does not require dispersing the substrate in water or the like. However, there are problems in the form of low sensitivity, the need for large quantities of enzyme solution, and difficulty in achieving high throughput.

According, the present invention has for its object to provide a new method for measuring the activity of an enzyme on a substrate that is insoluble or of low solubility in water. In particular, the present invention has for its object to provide a new method for measuring the activity of an enzyme on a substrate that is insoluble or of low solubility in water, permitting real-time measurement with a small quantity of enzyme, high sensitivity and a rapid reaction.

The present inventors discovered that pitting formed in the course of adding an enzymatic solution dropwise to the surface of a gel-like solid comprising a substrate that was insoluble or of low solubility in water as a dispersoid and water as a dispersion medium as needed. They further discovered that by measuring the volume of the pits, it was possible to highly sensitively and rapidly evaluate in real time and with high throughput the activity of an enzyme acting on a substrate that was insoluble or of low solubility in water using an extremely small quantity of enzyme solution. The present invention was devised on that basis.

The present invention makes it possible to quickly measure the activity of an enzyme on a substrate that is insoluble or of low solubility in water with high sensitivity, in real time, and with high throughput, even with a quantity of enzyme solution on the picoliter level.

### [Brief Description of the Drawings]

[Figure 1] Figure 1 shows a two-dimensional image (the number in the upper left is the quantity of solution dripped; the scale bar is 50 µm) and three-dimensional image (on right; scale bar is 200 µm) of pitting formed on the surface of a gel body surface.
[Figure 2] Figure 2 shows the correlation between the volume of pitting and the quantity of cellulase solution dripped onto the gel body.
[Figure 3] Figure 3 shows the change over time of the volume of pitting.

### [Modes of Carrying Out the Invention]

The method for measuring the activity of an enzyme of the present invention comprises:
(1) positioning a prescribed quantity of an enzyme on a portion of the surface of a gel body at least part of the dispersoid of which is comprised of the substrate of the enzyme;
(2) measuring the pits formed on the surface of the gel body by the action of the enzyme; and
(3) calculating the activity of the enzyme based on the measurement results and the prescribed quantity.

### <The enzyme that is evaluated>

The enzyme that is the subject of measurement in the method of the present invention has an action substrate that is insoluble or of low solubility in water. In the present invention, the phrase "insoluble or of low solubility in water" means solubility in 25°C water of 1 mg/mL or less. This includes substrates that were originally soluble in water but that have been rendered insoluble by an operation such as chemical crosslinking, physical crosslinking or the like.

Examples of the enzyme are cellulase, chitinase, agarase, mannanase, alginase, protease, esterase, and lipase. However, there is no intent to limit the target enzyme to these examples.

The substrate of the enzyme can be a polymer compound (such as a polysaccharide or protein), oil and fat. When the substrate is a polymer compound, from the perspective of insolubility or low solubility in water, it can be a polymer compound having a (number or weight) average molecular weight falling within a range of 10,000 to 5,000,000. However, this range is not intended to be a limitation. Many polymer compounds that are insoluble or of low solubility in water are compounds in which numerous groups are polymerized by glycoside bonds, ester bonds, amide bonds, ether bonds, or the like in the molecule. Accordingly, the enzyme can be one that hydrolyzes the substrate or breaks it down by oxidation. Oils and fats have ester bonds within the molecule. Lipase, the substrate of which is an oil and fat, is an enzyme that hydrolyzes the ester bonds of oils and fats.

**[Table 1]**

| | |
|---|---|
| Enzyme | Substrate |
| Cellulase | Cellulose |
| Chitinase | Chitin |
| Agarase | Agar |
| Mannanase | Polysaccharides, mannone |
| Alginase | Alginic acid |
| Protease | Protein |
| Esterase | Polyester |
| Lipase | Fats and oils |

In the method of the present invention, a "gel body at least part of the dispersoid of which is comprised of the substrate of the enzyme" is employed. From the perspective of permitting rapid measurement with high sensitivity, a "gel body the entirety of the dispersoid of which is comprised of the substrate of the enzyme" is desirably employed. In the present invention, the term "gel" refers to a state in which a dispersoid crosslinks to form a network, or a state in which a substrate is retained within the reticulations of a three-dimensional network comprised of a gelling agent. The method of crosslinking the dispersoid is not specifically limited. It can be covalent bonds, intermolecular forces, or both.

Examples of the substrate that is insoluble or of low solubility in water are polysaccharides (such as cellulose, chitin, and agar), proteins (such as albumen proteins and gelatins), and oils and fats (such as vegetable oils, animal oils, mineral oils, and lipids). However, there is no intent to limit the substrate to these. A gel body can be obtained by a method based on the type of substrate using these substrates as starting materials. Dispersoids can be incorporated in addition to the enzyme substrate, examples being 12-hydroxystearic acid, diisostearic acid metal salts, and other oil gelling agents. For example, when the substrate is an oil or fat, these oil gelling agents can be employed to form the dispersoid of a gel body. Although depending on the oil or fat, the addition of 0.1 to 10 mass% of an oil gelling agent to an oil or fat will form the dispersoid of a gel body suited to the method of the present invention.

In the gel body, the dispersoid is comprised of the enzyme substrate. The pores of the gel body can be filled with a gas such as air, or filled with water or a buffer. However, the gel body in which at least a part of the dispersoid is comprised of the substrate that is employed in the method of the present invention is desirably a gel body or a hydrogel body containing a dispersion medium in the form of water. Thus, the reaction of a small quantity of substrate with the enzyme by, for example, hydrolysis, yields a large change in volume, enhancing the pitting detection sensitivity and measurement precision.

To enhance sensitivity and the reaction rate, the substrate (dispersoid) content in the gel body is desirably low. However, when it becomes excessively low, there are problems in that the mechanical strength of the gel body diminishes, handling becomes difficult, and the impact of water present on the surface of the gel body precludes the enzyme solution from dripping properly. When the substrate (dispersoid) content becomes excessively high, there are problems in that preparation of the gel body becomes difficult, the reaction speed slows down, and evaporation of moisture in activity evaluation causes the surface of the substrate to deform, making it difficult to quantify the pitting.

In the present invention, a gelled solid comprising for example from 0.1 weight% to 30 mass%, preferably 0.5 mass% to 20 mass%, of substrate as a mass concentration can be properly employed as the reaction substrate. The shape of the gelled solid employed in the present invention is not limited, but since the lower detection limit of the pitting is specified by the roughness of the substrate surface, to increase detection sensitivity, the root mean square roughness of the gel body surface comprised of the substrate is desirably 2 µm or less, for example, preferably falling within a range of 0.1 to 1.0 µm.

When the substrate is cellulose, the gel body can be, for example, a gel-like structure body in which the dispersoid is cellulose molecules, prepared according to the method described in Patent Reference 1 or Nonpatent Reference 2, or bacterial cellulose.

When the substrate is chitin, the gel body can be, for example, a gel-like structure body in which the dispersoid is chitin molecules, prepared according to Nonpatent Reference 3, for example.

When the substrate is agar or gelatin, the gel body can be an agar gel or gelatin gel that is prepared, for example, by dissolving commercial agar (made by Wako Pure Chemical Industries, Ltd.) or commercial gelatin (made by Wako Pure Chemical Industries, Ltd.) in hot water, and then cooling it to room temperature and solidifying it.

When the substrate is an albumen protein, the gel body can be, for example, a gel-like structure body comprised of albumen protein gel that is prepared, for example, by boiling a chicken egg in a water bath for 15 minutes.

A prescribed quantity of the enzyme is positioned on part of the surface of the gel body. The prescribed quantity of enzyme that is positioned can be suitably determined taking into account the type of enzyme and substrate, the required reaction time, and the like. A prescribed quantity of enzyme is positioned, that is, any quantity can be employed. By using a predetermined quantity, the amount of degradation or the like of the substrate that is achieved with the prescribed quantity of enzyme can be calculated based on the results of measuring the pitting in a subsequent step. As a result, the activity of the enzyme can be calculated. Here, the "activity of the enzyme" means the amount of reaction of a unit quantity of enzyme with the substrate per unit time. The unit quantity of enzyme can be the mass of the enzyme or the volume of solution containing the enzyme. When it is the volume of the solution containing the enzyme, the enzymatic activity per mass of enzyme can also be calculated when the content of the enzyme in the solution containing the enzyme is known.

The enzyme can be positioned on the surface of the gel body by, for example, dripping or spraying the solution containing the enzyme. The quantity of the enzyme-containing solution is not specifically limited, and can be suitably selected based on the activity and concentration of the enzyme. For example, it can fall within a range of 1 pL to 1 nL. The lower limit of the quantity that is dripped or sprayed can be decreased based on the detection sensitivity of the pitting that is produced by the effect of the enzyme following dripping or spraying. It can also be set to 1 pL. There is no specific upper limit to the quantity that is dripped or sprayed. From the perspective of achieving rapid and real-time measurement, the quantity that is dripped or sprayed is desirably a relatively small quantity. A quantity of about 10 pL permits measurement with suitably high sensitivity. From the perspective of highly sensitive, rapid, real-time, and high throughput measurement, the quantity of enzyme-containing solution that is dripped or sprayed desirably falls within a range of 1 pL to 1 nL, preferably within a range of 1 pL to 100 pL. The enzymatic reaction can be conducted at room temperature. Based on the activity of the targeted enzyme, it can also be conducted with cooling or with heating. The temperature range of the enzymatic reaction can fall within a range of 0 to 100°C, for example.

### <The measurement method>

In the method for measuring enzymatic activity of the present invention, the degree to which the reaction progresses can be measured by measuring the pitting that is formed on the surface of the gel body by enzymatic hydrolysis. Examples of pitting measurement methods are methods of measuring the shape and volume of the pits, the method of measuring change in the light transmittance of the gel body accompanying the formation of pitting, and the method of measuring change in the light reflectance of the gel body surface accompanying the formation of pitting. However, the method that is used to measure pitting is not intended to be limited to these methods. The pitting that has formed on the surface of the gel body can be measured after positioning the enzyme, over time, or after a certain amount of time has elapsed following positioning of the enzyme. The duration of the enzymatic reaction can be suitably determined based on the type of enzyme, the type (for example, the molecular weight) or concentration (content) of substrate constituting the gel body, the measurement temperature, and the like. For example, it can be set within a range of from 1 minute to 48 hours. However, there is no intent to limit it to this range. Depending on the conditions, there will sometimes be cases where the enzymatic activity can be measured for a period of less than 1 minute. It is also possible to measure the enzymatic activity for a period exceeding 48 hours.

The shape and volume of the pitting can be measured, for example, using a 3D laser microscope. In addition to a 3D laser microscope, a scanning probe microscope, optical interference-type profilometer, contact-type surface roughness measuring apparatus, or the like can be used to measure the shape and volume of the pitting. The method of measuring change in the light transmittance of the gel member accompanying the formation of pitting can be implemented, for example, using a spectrophotometer or an optical sensor (such as a photomultiplier tube or photodiode). The method of measuring change in the light reflectance of the gel body surface accompanying the formation of pitting can be implemented, for example, by irradiating the surface of the gel member with light and measuring the intensity of the reflected light with a spectrophotometer or an optical sensor (such as a photomultiplier tube or a photodiode).

### <Method of calculating activity>

In the method for measuring enzymatic activity of the present invention, the enzymatic activity is calculated based on the results of measuring the pitting. For example, when employing the measurement results obtained by the method of measuring the shape and volume of the pitting, the density of the dispersoid of the gel body is obtained in advance, and from this density and the volume, the mass or the like of the substrate (dispersoid) that has been degraded by the enzyme is calculated to calculate the enzymatic activity from the quantity of enzyme employed and the reaction duration.

When employing measurement results obtained by the method of measuring the change in the light transmittance of the gel body accompanying the formation of pitting, the presence of enzymatic activity or its strength can be qualitatively detected from the change in the light transmittance, for example. Further, for example, the shape and volume of pitting, and the change in the light transmittance accompanying this, can be determined in advance and a calibration curve plotted to make it possible to calculate the enzymatic activity from the quantity of enzyme employed and the reaction duration by calculating the mass or the like of the substrate (dispersoid) that has been degraded by the enzyme from change in the level of light transmittance.

When employing measurement results obtained by the method of measuring change in the optical reflectance of the surface of the gel body accompanying the formation of pitting, the presence of enzymatic activity and its strength can be qualitatively detected, for example, from change in the optical reflectance. Further, for example, the shape and volume of the pitting, and the change in the optical reflectance accompanying this, can be obtained in advance and a calibration curve plotted to make it possible to calculate the enzymatic activity from the quantity of enzyme employed and the reaction duration by calculating the mass or the like of the substrate (dispersoid) that has been degraded by the enzyme from change in the level of optical reflectance.

The present invention will be described in greater detail below through embodiments.

### <The enzyme>

Meicelase made by Meiji Seika (derived from *Trichoderma viride*) and celluclast made by Novozyme (derived from *Trichoderma reesei*) were employed as cellulase.

Chitinase (derived from *Streptomyces griseus*) and agarase (derived from *Pseudomonas atlantica*) were purchased from Sigma Aldrich and protease (Protease K) was purchased from Wako Pure Chemical Industries, Ltd.

### <Preparation of gel body (gel)>

Cellulose gel was prepared according to the description in Nonpatent Reference 2 and immersed in 0.1 M acetate buffer (pH 5.2). Chitin gel was prepared according to the description in Nonpatent Reference 3 and immersed in 0.1 M acetate buffer (pH 5.2). Agar gel was prepared by dissolving commercial agar (made by Wako Pure Chemical Industries, Ltd.) in hot water, and cooling the solution to room temperature to solidify it. Albumen protein gel was prepared by boiling a chicken egg in a water bath for 15 minutes. Gelatin gel was prepared by dissolving a commercial gelatin (made by Wako Pure Chemical Industries, Ltd.) in hot water, and cooling the solution to room temperature to solidify it. Bacterial cellulose was prepared by rinsing commercial Nata de Coco (made by Izu Fermente Co., Ltd.) in hot water, immersing it in 0.1 M acetate buffer (pH 5.2), and drying it to the point where no water was released at room temperature.

### <Dripping enzyme solution>

A LaboJet-500 bio (made by Microjet K.K.) was employed to drip the enzyme solution. A 10 pL quantity of solution was dripped each time. Repeated dripping was conducted in a single location to control (increase/decrease) the total quantity of solution being dripped on the gel body.

### <Quantifying enzymatic activity>

After dripping the enzyme solution, the volume of the pitting that had been formed on the surface of the gel body by hydrolysis was measured with a VK-9700 Generation II (made by Keyence Corp.). The software that came with the device was used to calculate the volume. The mass of the substrate that had been degraded was calculated from the volume data obtained and the concentration of the substrate contained in the gel body, and the enzymatic activity was then quantified from the quantity of enzyme employed and the reaction duration.

### Embodiment 1: Evaluation of cellulase activity

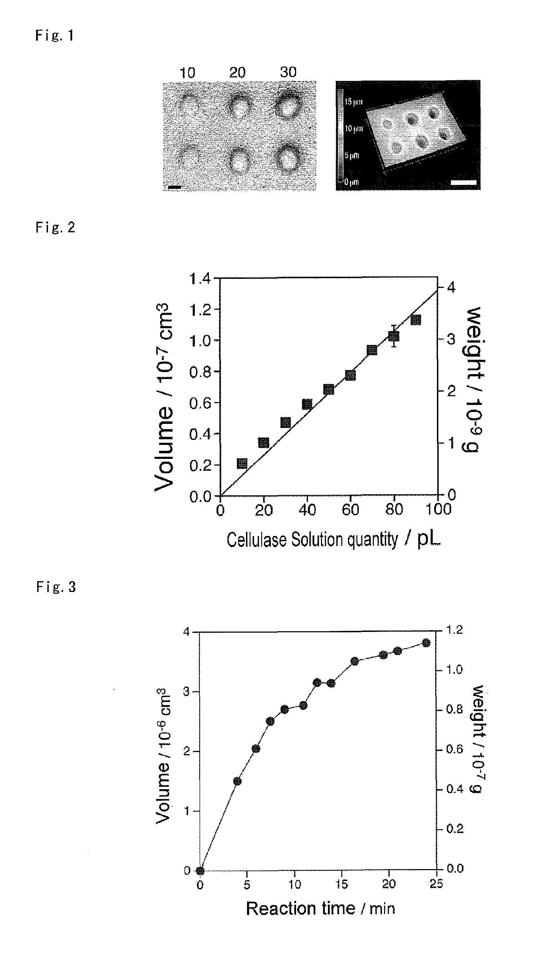

Meicelase solutions (10 mg/mL) of 10, 20, 30, 40, 50, 60, 70, 80, and 90 pL were dripped at an equal 200 µm spacing on the surface of a gel containing 3 mass% cellulose. A reaction was conducted for 30 minutes at room temperature, after which the pitting that had been produced on the surface of the gel body by enzymatic hydrolysis was observed by 3D laser microscopy and measured (Figure 1). The mass of the degraded cellulose was calculated from the density (30 mg/mL) of the gel body.

It was found that extremely minute changes in volume of about 10⁻⁷cm³ due to enzymatic hydrolysis could be measured well by measuring the volume of the pitting (Figure 2). The volume of the pitting was proportionate to the quantity of enzyme solution that had been dripped, and so could be used as a quantitative indicator in enzymatic hydrolysis evaluation of the pitting. The mass of the cellulose that had been degraded accompanying the formation of pitting was estimated at several picograms, permitting the detection with ultra-high sensitivity of the enzymatic hydrolysis of extremely small quantities of cellulose. The present method was also found to permit the analysis of 100 reactions on a gel body measuring about 2 cm square. The enzymatic activity of meicelase (cellulase) was calculated to be 0.12 to 0.21 mg cellulose/mg enzyme/minute.

### Embodiment 2: Evaluation of cellulase activity

A 1 nL meicelase solution (10 mg/mL) was dripped onto the surface of a gel containing 3 mass% cellulose. While conducting the reaction at room temperature, the volume of the pitting that was produced on the surface of the gel body by enzymatic hydrolysis were measured over time. The mass of the cellulose that was degraded was calculated from the density of the gel body (30 mg/mL).

The volume of the pitting increased over time, becoming nearly constant at about 15 minutes after the start of the reaction (Figure 3). The volume of the pitting formed was about 10⁻⁶ cm³, and the mass of the cellulose that had been degraded in the process was about 100 ng. Using a small 1 nL quantity of enzyme solution, it was found possible to track the degree of progression of the enzyme hydrolysis reaction due to cellulase with ultra-high sensitivity and in real time. The enzymatic activity of meicelase was calculated to be 1.01 mg cellulose/mg enzyme/minute.

### Embodiment 3: Evaluation of cellulase activity

Meicelase solutions (10 mg/mL) of 20, 40, 60, 80, and 100 pL were dripped at an equal 300 µm spacing on the surface of a gel containing 1 mass% cellulose. A reaction was conducted for 14 minutes at room temperature, after which the pitting that had been produced on the surface of the gel body by enzymatic hydrolysis was quantified. The mass of the degraded cellulose was calculated from the density (10 mg/mL) of the gel body.

It was found that extremely minute changes in volume of about 5.9 x 10⁻⁸ to 2 x 10⁻⁷ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the cellulose that had been degraded accompanying the formation of pitting was 0.6 to 2 ng. As a result, the enzymatic activity of meicelase (cellulase) was calculated to be 0.13 to 0.21 mg cellulose/mg enzyme/minute.

### Embodiment 4: Evaluation of cellulase activity

Meicelase solutions (10 mg/mL) of 20, 40, 60, 80, and 100 pL were dripped at an equal 300 µm spacing on the surface of a gel containing 10 mass% cellulose. A reaction was conducted for 13 minutes at room temperature, after which the pitting that had been produced on the surface of the gel body by enzymatic hydrolysis was quantified. The mass of the degraded cellulose was calculated from the density (100 mg/mL) of the gel body.

It was found that extremely minute changes in volume of about 4.4 x 10⁻⁸ to 1.2 x 10⁻⁷ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the cellulose that had been degraded accompanying the formation of pitting was 4.4 to 12.0 ng. As a result, the enzymatic activity of meicelase (cellulase) was calculated to be 0.92 to 1.71 mg cellulose/mg enzyme/minute.

### Embodiment 5: Evaluation of cellulase activity

Celluclast solutions (commercial solutions diluted two-fold) of 10, 20, 40, 60, 80, and 100 pL were dripped at an equal 400 µm spacing on the surface of a gel containing 3 mass% cellulose. A reaction was conducted for 6 minutes at room temperature, after which the pitting that had been produced on the surface of the gel body by enzymatic hydrolysis was quantified. The mass of the degraded cellulose was calculated from the density (30 mg/mL) of the gel body.

It was found that extremely minute changes in volume of about 3.6 x 10⁻⁸ to 2.1 x 10⁻⁷ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the cellulose that had been degraded accompanying the formation of pitting was estimated to be 1.1 to 6.2 ng, and enzymatic hydrolysis of extremely small amounts of cellulose was detected with ultra-high sensitivity. As a result, the enzymatic activity of celluclast (cellulase) was calculated to be 10.41 to 17.88 mg cellulose/mL enzyme solution/minute.

### Embodiment 6: Evaluation of chitinase activity

Chitinase solutions (8 mg/mL) of 10, 20, 40, and 100 pL were dripped at an equal 400 µm spacing on the surface of a gel containing 3 mass% chitin. A reaction was conducted for 3 minutes at room temperature, after which the pitting that had been produced on the surface of the gel body by enzymatic hydrolysis was quantified. The mass of the degraded chitin was calculated from the density (30 mg/mL) of the gel body.

It was found that extremely minute changes in volume of about 6.8 x 10⁻⁸ to 2.2 x 10⁻⁷ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the chitin that had been degraded accompanying the formation of pitting was estimated to be 2.0 to 6.7 ng, and enzymatic hydrolysis of extremely small amounts of chitin was detected with ultra-high sensitivity. As a result, the enzymatic activity of chitinase was calculated to be 2.7 to 8.5 mg chitin/mg enzyme/minute.

### Embodiment 7: Evaluation of agarase activity

An 80 pL agarase solution (0.4 mg/mL) was dripped on the surface of a gel containing 1 mass% agar. A reaction was conducted for 120 minutes at room temperature, after which the pitting that had been produced on the surface of the gel body by enzymatic hydrolysis was quantified. The mass of the degraded agar was calculated from the density (10 mg/mL) of the gel body.

It was found that an extremely minute change in volume of about 2 x 10⁻⁸ cm³ due to enzymatic hydrolysis could be measured well by measuring the volume of the pitting. The mass of the agar that had been degraded accompanying the formation of pitting was estimated to be 200 pg, and enzymatic hydrolysis of extremely small amounts of agar was detected with ultra-high sensitivity. As a result, the enzymatic activity of agarase was calculated to be 0.05 mg agar/mg enzyme/minute.

### Embodiment 8: Evaluation of protease activity

Protease solutions (20 mg/mL) of 60 and 80 pL were dripped at an equal 400 µm spacing on the surface of a gel containing 12 mass% albumen protein. A reaction was conducted for 15 minutes at room temperature, after which the pitting that had been produced on the surface of the gel body by enzymatic hydrolysis was quantified. The mass of the degraded albumen protein was calculated from the density (120 mg/mL) of the gel body.

It was found that extremely minute changes in volume of about 3.1 x 10⁻⁷ to 5.5 x 10⁻⁷ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the albumen protein that had been degraded accompanying the formation of pitting was estimated to be 36.6 to 66.2 ng, and enzymatic hydrolysis of extremely small amounts of albumen protein was detected with ultra-high sensitivity. As a result, the enzymatic activity of protease was calculated to be 2.03 to 2.76 mg albumen protein/mg enzyme/minute.

### Embodiment 9: Evaluation of protease activity

Protease solutions (2 mg/mL) of 10, 40, 60 and 80 pL were dripped at an equal 500 µm spacing on the surface of a gel containing 10 mass% gelatin. A reaction was conducted for 5 minutes at room temperature, after which the pitting that had been produced on the surface of the gel body by enzymatic hydrolysis was quantified. The mass of the degraded gelatin was calculated from the density (100 mg/mL) of the gel body.

It was found that extremely minute changes in volume of about 7.9 x 10⁻⁹ to 5.5 x 10⁻⁸ cm³ due to enzymatic hydrolysis could be measured well by measuring the volume of the pitting. The volume of the pitting was proportionate to the quantity of enzyme solution that had been dripped, and so could be used as a quantitative indicator in enzymatic hydrolysis evaluation of the pitting. The mass of the gelatin that had been degraded accompanying the formation of pitting was estimated to be 0.7 to 5.5 ng, and enzymatic hydrolysis of extremely small amounts of gelatin was detected with ultra-high sensitivity. As a result, the enzymatic activity of protease was calculated to be 6.12 to 6.92 mg gelatin/mg enzyme/minute.

### Embodiment 10: Evaluation of cellulase activity

A meicelase solution (10 mg/mL) of 60 pL was dripped on the surface of a gel containing 1 mass% bacterial cellulose. A reaction was conducted for 11 minutes at room temperature, after which the pitting that had been produced on the surface of the gel body by enzymatic hydrolysis was quantified. The mass of the degraded bacterial cellulose was calculated from the density (10 mg/mL) of the gel body.

It was found that an extremely minute change in volume of about 1.9 x 10⁻⁸ cm³ due to enzymatic hydrolysis could be measured well by measuring the volume of the pitting. The mass of the bacterial cellulose that had been degraded accompanying the formation of pitting was estimated at 190 pg and enzymatic hydrolysis of an extremely small amount of bacterial cellulose was detected with ultra-high sensitivity. As a result, the enzymatic activity of meicelase (cellulase) was calculated to be 0.03 mg cellulose/mg enzyme/minute.

### [Industrial Applicability]

The present invention is useful in fields relating to enzymes, particularly enzymes with substrates that are insoluble or of low solubility in water.

## Claims

1. A method of measurement of activity of an enzyme comprising:
positioning a prescribed quantity of an enzyme on a portion of the surface of a gel body at least part of the dispersoid of which is comprised of the substrate of the enzyme;
measuring the pits formed on the surface of the gel body by the action of the enzyme; and
calculating the activity of the enzyme based on the measurement results and the prescribed quantity.

2. The method according to claim 1, wherein the substrate of the enzyme is a material being insoluble or of low solubility in water.

3. The method according to claim 2, wherein the substrate of the enzyme is a polymer compound having a number or weight average molecular weight falling within a range of 10,000 to 5,000,000.

4. The method according to claim 2 or 3, wherein the gel body contains water as a dispersion medium.

5. The method according to any one of claims 1-4, wherein the enzyme is an enzyme hydrolyzing the substrate or an enzyme breaking the substrate down by oxidation.

6. The method according to any one of claims 1-5, wherein the positioning of the enzyme on the surface of the gel body is conducted by dripping or spraying the solution containing the enzyme.

7. The method according to claim 6, wherein the quantity of the enzyme-containing solution falls within a range of 1 pL to 1 nL.

8. The method according to any one of claims 1-7, wherein the measurement of the pitting that has formed on the surface of the gel body is conducted after positioning the enzyme, over time, or after a certain amount of time has elapsed following positioning of the enzyme.

9. The method according to any one of claims 1-8, wherein the pitting measurement method is a method of measuring the shape and volume of the pits, the method of measuring change in the light transmittance of the gel body accompanying the formation of pitting, or the method of measuring change in the light reflectance of the gel body surface accompanying the formation of pitting.

10. The method according to claim 9, wherein the volume of the pitting is measured by using a 3D laser microscope.

11. The method according to any one of claims 2-10, wherein the substrate being insoluble or of low solubility in water is a polysaccharide or a protein.

12. The method according to claim 11, wherein the polysaccharide is cellulose, chitin, or agar.

13. The method according to claim 11, wherein the protein is an albumen protein or gelatin.
